# EUROPEAN PATENT APPLICATION

(11) **EP 4 431 083 A1**
(43) Date of publication of application: **18.09.2024**
(21) Application number: 23382249.3
(22) Date of filing: 16.03.2023
(51) Int. Cl.: A61K 9/00, A61K 31/7042, A61K 47/40, A61P 31/04

(54) **FORMULATION OF DALBAVANCIN**

(71) Applicant: Adalvo Limited, San Gwann, SGN 3000 (MT)
(72) Inventor: CARRETO CANO, Francisco, Madrid (ES); SANCLIMENS PEREZ DE ROZAS, Gloria, Madrid (ES); STRUSI, Orazio Luca, San Gwann (MT)
(74) Representative: Slavík, Jiri

(57) **Abstract**

The present invention relates to a pharmaceutical formulation containing dalbavancin or a pharmaceutically acceptable salt thereof, a cyclodextrin and water. The novel fonnulations have a significantly increased stability over known dalbavancin formulations.

## Description

### Field of Art

The invention relates to a stable formulation of dalbavancin.

### Background Art

Dalbavancin is a lipoglycopeptide antibiotic. Dalvabancin has strong activity against many Grampositive bacteria, in particular methicillin-sensitive and methicillin-resistant *Staphylococcus aureus, Streptococcus pyogenes, Streptococcus agalactiae, Streptococcus anginosus, Streptococcus intermedius,* and *Streptococcus constellates.*

Dalbavancin (sold under the trade names Dalvance, Xydalba) is used to treat acute bacterial skin and skin structure infections, and it is administered in the form of an infusion solution. Dalbavancin has also been designated by the FDA for the treatment of acute osteomyelitis in children, as an orphan disease.

Xydalba or Dalvance are sold as lyophilized powders which need two steps for preparing the infusion solution, both steps need to be performed aseptically. A first step is a reconstitution. The reconstitution step is carried out by adding water for injections into a vial containing the lyophilized powder and gently swirling or inverting the vial, as there is a danger of foaming. The reconstitution time may be up to 5 minutes. The reconstituted concentrate must then be further diluted with 5% glucose solution for infusion. It may be challenging to perform the reconstitution step under aseptic conditions in any situation, and the reconstitution time is rather long and burdensome in hospitals. If the reconstitution is not performed correctly and the concentrate starts foaming, it is not suitable for further use.

The formulation of Xydalba or Dalvance contains dalbavancin, mannitol, lactose monohydrate, and hydrochloric acid and sodium hydroxide for pH adjustment. Mannitol acts as stabilizer for dabavancin in the solid phase. Chemical and physical in-use stability of Xydalba has been demonstrated for both the reconstituted concentrate and for the diluted solution for 48 hours at or below 25 °C.

WO 2004/045636, and to some extent also WO 2004/045637, relate to formulations of dalbavancin which correspond to the commercial product. The stabilizers listed in these patent applications include mannitol, lactose, sucrose, sorbitol, glycerol, cellulose, trehalose, maltose, raffinose or mixtures thereof. The present invention aims at providing a formulation of dalbavancin which would be sufficiently stable in liquid form so that the reconstitution step can be avoided.

### Summary of the Invention

The present invention provides a pharmaceutical formulation containing dalbavancin or a pharmaceutically acceptable salt thereof, a cyclodextrin and water.

Preferably, the cyclodextrin is selected from sulfobutyl ether beta-cyclodextrin (SBECD) and hydroxypropyl beta-cyclodextrin (HPBCD). SBECD is preferably present as a sodium salt. HPBCD is particularly preferred.

Preferably, the molar ratio of dalbavancin to cyclodextrin is within the range of from 1:0.1 to 1:6, more preferably 1:0.5 to 1:6, yet more preferably 1:2.5 to 1:6, even more preferably 1:3 to 1:5.

In some embodiments, the cyclodextrin is sulfobutyl ether beta-cyclodextrin or a sodium salt thereof, and the molar ratio of dalbavancin to cyclodextrin is within the range of from 1:2.5 to 1:6, even more preferably 1:3 to 1:5.

In some embodiments, the cyclodextrin is hydroxypropyl beta-cyclodextrin, and the molar ratio of dalbavancin to cyclodextrin is within the range of from 1:0.1 to 1:6, more preferably 1:0.5 to 1:6, even more preferably 1:1 to 1:5.

Dalbavancin is a semi-synthetic glycopeptide mixture as described in detail e.g. in EP 1565201 B2.

The pharmaceutically acceptable salt of dalbavancin may be a salt formed by a reaction of an organic acid or an inorganic acid. Such acids may be hydrochloric, hydrobromic, sulfuric, phosphoric, acetic, trifluoroacetic, trichloroacetic, succinic, citric, ascorbic, lactic, maleic, glutamic, camphoric, glutaric, glycolic, phthalic, tartaric, lauric, stearic, salicylic, methanesulfonic,benzenesulfonic, sorbic, picric, benzoic, cinnamic. The pharmaceutically acceptable salt of dalbavancin is preferably dalbavancin hydrochloride.

The pharmaceutically acceptable salt of dalbavancin may alternatively be formed with bases, preferably with alkali metal hydroxides, alkaline earth metal hydroxides, ammonia and amines. For example, the bases may include sodium hydroxide, potassium hydroxide, calcium hydroxide, magnesium hydroxide, baryum hydroxide, ammonia, methylamine, dimethylamine, diethylamine, ethanolamine, picoline.

Water used in the formulation is typically water for injections.

The formulation preferably has a pH within the range of 3 to 5.5, preferably 4 to 5.3, even more preferably about 4.5.

The pH may be adjusted, e.g., by addition of hydrochloride acid and sodium hydroxide.

The cyclodextrin formulation was found to be surprisingly stable in the order of months, in particular if the dalbavancin to cyclodextrin molar ratio is within the indicated range. Other candidate stabilizers, such as glucose (a representative of sugars), failed to stabilize the dalbavancin formulation.

In some embodiments, the formulation of the invention contains at least 95 % of the initial amount of dalbavancin or a pharmaceutically acceptable salt thereof after 1 month at 25 °C, and at least 90 %, preferably at least 95 % of the initial amount of dalbavancin or a pharmaceutically acceptable salt thereof after 3 months at 25 °C.

In some embodiments, the formulation of the invention contains at least 90 % of the initial amount of dalbavancin or a pharmaceutically acceptable salt thereof after 1 month at 40 °C, and at least 75 % of the initial amount of dalbavancin or a pharmaceutically acceptable salt thereof after 3 months at 40 °C. This temperature is not a temperature at which the product would be stored, but it is a temperature commonly used for accelerated stability study conditions.

In some embodiments, the formulation of the invention contains at least 97 % of the initial amount of dalbavancin or a pharmaceutically acceptable salt thereof after 1 month at 5 °C, and at least 96 % of the initial amount of dalbavancin or a pharmaceutically acceptable salt thereof after 3 months at 5 °C.

The reference method for measurement of the content of dalbavancin or a pharmaceutically acceptable salt thereof is HPLC (more specifically, reverse phase HPLC).

The pharmaceutical formulation of the invention is preferably free of sugars (monosaccharides, disaccharides) and sugar alcohols, in particular free of mannitol and lactose.

The pharmaceutical formulation according to the present invention can be as a concentrate for an infusion solution or parenteral solution. The pharmaceutical formulation according to the present invention can be an infusion solution or a parenteral solution (i.e. ready-to-use solution). The infusion or parenteral solution may be formed from the concentrate by dilution with a glucose solution (e.g. 5% (w/v) glucose solution) or with water for infusions.

The concentrate may contain dalbavancin or a pharmaceutically acceptable salt thereof in a concentration of 1 to 100 mg per ml of concentrate, preferably 10 to 70 mg per ml of concentrate, even more preferably 20 to 60 mg per ml of concentrate.

Typically, the formulation contains dalbavancin or a pharmaceutically acceptable salt thereof in a concentration of 0.1 to 100 mg per ml of final solution, preferably 0.5 to 50 mg per ml of final solution, or 1 to 10 mg per ml of final solution.

The formulation of the invention may be administered by infusion or by parenteral injection. It may be administered to prevent a bacterial infection or to treat a bacterial infection. The treatment may be performed once or repeated as needed. Typical delays between individual administrations may range between 1 and 14 days, more preferably between 5 and 9 days.

Bacterial infection may be an infection caused, for example, by Staphylococcus (incl. *Staphylococcus aureus*), *Neisseria, Clostridium, Streptococcus* (incl. *Streptococcus pyogenes, Streptococcus agalactiae, Streptococcus anginosus, Streptococcus intermedius,* and *Streptococcus constellates*) species.

The formulation of the invention may be co-administered with another antibiotic. The co-administration may be sequential or simultaneous. Sequential co-administration refers to administering dalbavancin in another time point than another antibiotic. Simultaneous co-administration refers to administering dalbavancin at the same time point as another antibiotic. In simultaneous co-administration, dalbavancin and another antibiotic may be administered together in one formulation or they may be administered in two (or more) separate formulations, at the same time.

The formulation of the invention may be also used in the treatment of acute osteomyelitis in children.

### Examples

### Example 1: Formulations

The composition of the formulations in mg/ml are shown in the following table:

| Preparation | Dalbavancin HCl (mg/ml) | HPBCD (mg/ml) | SBECD (mg/ml) | Molar ratio (Dal:CD) | NaOH (1N) | HCl (0.1N) | Water for injection |
|---|---|---|---|---|---|---|---|
| AD03-1 (control) | 20.64 | - | - | - | q.s. pH 4.2 | q.s. pH 4.2 | q.s. 1 ml |
| AD03-2 (comparative) | 20.64 | glucose: 366.67 | | *n.a.* | q.s. pH 4.2 | q.s. pH 4.2 | q.s. 1 ml |
| AD03-3 | 20.64 | - | 23.82 | 1:1 | q.s. pH 4.5 | q.s. pH 4.5 | q.s. 1 ml |
| AD03-4 | 20.64 | - | 71.45 | 1:3 | q.s. pH 5.3 | q.s. pH 5.3 | q.s. 1 ml |
| AD03-5 | 20.64 | - | 119.08 | 1:5 | q.s. pH 4.5 | q.s. pH 4.5 | q.s. 1 ml |
| AD03-6 | 51.605 | - | 59.54 | 1:1 | q.s. pH 4.5 | q.s. pH 4.5 | q.s. 1 ml |
| AD03-7 | 51.605 | - | 178.62 | 1:3 | q.s. pH 4.5 | q.s. pH 4.5 | q.s. 1 ml |
| AD03-8 | 51.605 | - | 297.69 | 1:5 | q.s. pH 4.5 | q.s. pH 4.5 | q.s. 1 ml |
| AD03-9 | 20.642 | 15.412 | - | 1:1 | q.s. pH 4.5 | q.s. pH 4.5 | q.s. 1 ml |
| AD03-10 | 20.642 | 46.237 | - | 1:3 | q.s. pH 5.0 | q.s. pH 5.0 | q.s. 1 ml |
| AD03-11 | 20.642 | 77.062 | - | 1:5 | q.s. pH 4.5 | q.s. pH 4.5 | q.s. 1 ml |
| AD03-12 | 51.605 | 38.527 | - | 1:1 | q.s. pH 4.5 | q.s. pH 4.5 | q.s. 1 ml |
| AD03-13 | 51.605 | 115.582 | - | 1:3 | q.s. pH 4.5 | q.s. pH 4.5 | q.s. 1 ml |
| AD03-14 | 51.605 | 192.637 | - | 1:5 | q.s. pH 4.5 | q.s. pH 4.5 | q.s. 1 ml |
| AD03-15 | 20.64 | - | 71.45 | 1:3 | q.s. pH 4.5 | q.s. pH 4.5 | q.s. 1 ml |
| AD03-16 | 20.642 | 46.237 | - | 1:3 | q.s. pH 4.5 | q.s. pH 4.5 | q.s. 1 ml |

The cyclodextrin formulations were prepared by the following procedure: 80% of the total volume was poured into the beaker. The cyclodextrin was added and the beaker was shaken until complete dissolution of the cyclodextrin. Dalbavancin hydrochloride was added gently, avoiding the formation of foam. The pH value was adjusted by addition of NaOH or HCl solution as needed. The solution was made up to volume in a volumetric flask by addition of water. The product was shaken for 10 minutes.

### Example 2: Stability testing assay

In this example, the dalbavancin content (%) at the start (T0), after 1 month (T1), after 2 months (T2) and after 3 months (T3) was measured under the conditions indicated in the table (temperature, relative humidity).

Vials containing 5 ml of formulations prepared according to example 1 were stored at temperatures described below over a period of 1 month (T1), 2 months (T2) and 3 months (T3).
5°C ± 3°C (conditions for storage in a refrigerator)
25°C ± 2°C / 60% RH ± 5% RH (long term stability study conditions)
40°C ± 2°C / 75% RH ± 5% RH (accelerated stability study conditions)

Freshly prepared formulations and formulations stored for 1, 2 and 3 months, respectively, were tested for dalbavancin content. The dalbavancin content was determined using HPLC. The HPLC column used was Ultimate XB-C18 (4.6 mm x 250 mm x 5 µm). Sample solvent was water : acetonitrile (70:30 v/v), column temperature 50 °C, flow rate 1.0 mL/min. 10 microliters of sample were injected on each run. Mobile phase was A: 0.025 mol/L aqueous sodium dihydrogen phosphate : acetonitrile (90:10), B: 0.025 mol/L aqueous sodium dihydrogen phosphate : acetonitrile (30:70). Gradient of mobile phase was from 67%A and 33%B to 30%A and 70%B, and then back to 67%A and 33%B. Detection was performed by UV/VIS detector at 280 nm.

The table in example 2 shows the relative dalbavancin content in the stored formulation, relative to the nominal amount of dalbavancin used for preparation of the formulation, expressed as a percentage.

| Preparation | **T0** | **T1** | | | **T2** | | | **T3** | | |
|---|---|---|---|---|---|---|---|---|---|---|
| | -20°C | 5°C | 25°C | 40°C | 5°C | 25°C | 40°C | 5°C | 25°C | 40°C |
| | | | 60% | 75% | | 60% | 75% | | 60% | 75% |
| | | | RH | RH | | RH | RH | | RH | RH |
| AD03-1 (control) | 101.6 | -- | -- | 92.7 | -- | -- | 81.0 | -- | -- | 64.5 |
| AD03-02 (comparati ve) | 100.8 | -- | -- | 85.7 | -- | -- | 76.2 | -- | -- | 62.7 |
| AD03-3 | prec. | - | - | - | - | - | - | - | - | - |
| AD03-4 | 109.3 | 99.5 | 97.4 | 96.7 | 104.1 | 102.3 | 95.4 | 97.0 | 97.9 | 85.6 |
| AD03-5 | 101.5 | 98.9 | 99.1 | 94.8 | 103.4 | 101.5 | 91.9 | 97.3 | 99.0 | 84.4 |
| AD03-6 | prec. | - | - | - | - | - | - | - | - | - |
| AD03-7 | 104.0 | 100.3 | 99.9 | 93.0 | 104.6 | 101.9 | 89.0 | 97.2 | 98.7 | 77.9 |
| AD03-8 | 99.7 | 101.0 | 98.0 | 88.6 | 104.4 | 101.3 | 88.2 | 98.0 | 98.0 | 77.3 |
| AD03-9 | 101.1 | 99.9 | 98.0 | 98.9 | 103.1 | 108.7 | 96.0 | 97.0 | 98.9 | 86.3 |
| AD03-10 | 101.0 | 101.5 | 99.1 | 95.8 | 102.2 | 101.4 | 95.2 | 97.4 | 97.2 | 86.5 |
| AD03-11 | 101.8 | 99.5 | 101.3 | 98.0 | 103.3 | 101.7 | 94.3 | 97.7 | 98.1 | 84.5 |
| AD03-12 | 100.6 | 98.9 | 99.7 | 99.5 | 103.9 | 101.4 | 92.8 | 97.6 | 97.9 | 84.1 |
| AD03-13 | 100.0 | 98.5 | 100.1 | 93.9 | 102.3 | 100.7 | 90.8 | 97.3 | 98.5 | 78.5 |
| AD03-14 | 108.8 | 98.9 | 100.4 | 83.0 | 102.7 | 101.1 | 89.9 | 97.9 | 97.0 | 79.3 |
| AD03-15 | 101.3 | 100.0 | 98.7 | 95.5 | 102.1 | 101.3 | 91.2 | 99.2 | 97.9 | 79.1 |
| AD03-16 | 109.2 | 100.8 | 97.7 | 96.6 | 102.9 | 102.0 | 95.6 | 99.7 | 98.2 | 85.9 |

| | | | | | | | | | | |
|---|---|---|---|---|---|---|---|---|---|---|
| Compositions AD03-3 and AD03-6 were not assayed because precipitates formed immediately after preparation of the compositions. | | | | | | | | | | |

### Example 3: Stability testing (impurities)

In this example, the total impurities content (%) at the start (T0), after 1 month (T1), after 2 months (T2) and after 3 months (T3) was measured under the conditions indicated in the table (temperature, relative humidity).

Vials containing 5 ml of formulations prepared according to example 1 were stored at temperatures described below over a period of 1 month (T1), 2 months (T2) and 3 months (T3).
5°C ± 3°C (conditions for storage in a refrigerator)
25°C ± 2°C / 60% RH ± 5% RH (long term stability study conditions)
40°C ± 2°C / 75% RH ± 5% RH (accelerated stability study conditions)

Freshly prepared formulations and formulations stored for 1, 2 and 3 months, respectively, were tested for the content of impurities (related substances). The content of impurities was determined using HPLC. The HPLC column used was Phenomenex Aeris Peptide XB-C18 (4.6 mm x 250 mm x 3.6 µm) and Waters Xbridge Peptide BEH C18 (4.6X150mm, 3.5 µm), combined in series. Sample solvent was water : acetonitrile (70:30 v/v), column temperature 50 °C, flow rate 1.0 mL/min. 10 microliters of sample were injected on each run. Mobile phase was A: 50mM aqueous ammonium dihydrogen phosphate (pH 5.5) : acetonitrile (95:5), B: 50mM aqueous ammonium dihydrogen phosphate (pH 5.5) : acetonitrile (40:60). Gradient of mobile phase was from 70%A and 30%B to 40%A and 60%B, and then back to 70%A and 30%B. Detection was performed by UV/VIS detector at 220 nm.

The amount of impurities is shown as weight percentage, relative to the nominal amount of dalbavancin used for preparation of the formulation.

| Preparation | **T0** | **T1** | | | **T2** | | | **T3** | | |
|---|---|---|---|---|---|---|---|---|---|---|
| | -20°C | 2-8°C | 25°C | 40°C | 2-8°C | 25°C | 40°C | 2-8°C | 25°C | 40°C |
| | | | 60% | 75% | | 60% | 75% | | 60% | 75% |
| | | | RH | RH | | RH | RH | | RH | RH |
| AD03-1 (control) | - | - | - | 9.2 | - | - | 11.6 | - | - | 16.9 |
| AD03-2 (comparative) | - | - | - | 11.0 | - | - | 14.3 | - | - | 21.7 |
| AD03-3 | prec. | - | - | - | - | - | - | - | - | - |
| AD03-4 | 1.1 | 1.05 | 1.6 | 4.9 | 0.7 | 1.2 | 5.4 | 0.8 | 1.6 | 8.5 |
| AD03-5 | 1.1 | 1.10 | 1.9 | 6.0 | 0.7 | 1.2 | 6.6 | 0.9 | 1.8 | 10.3 |
| AD03-6 | prec. | - | - | - | - | - | - | - | - | - |
| AD03-7 | 1.0 | 1.09 | 1.9 | 7.2 | 0.7 | 1.5 | 8.3 | 0.9 | 2.3 | 13.3 |
| AD03-8 | 1.0 | 1.09 | 1.9 | 7.8 | 0.7 | 1.5 | 9.1 | 0.9 | 2.4 | 14.3 |
| AD03-9 | 1.4 | 1.60 | 1.5 | 3.6 | 0.7 | 1.0 | 4.7 | 0.7 | 1.5 | 7.5 |
| AD03-10 | 1.1 | 1.15 | 1.5 | 4.2 | 0.8 | 1.1 | 5.5 | 0.9 | 1.8 | 9.0 |
| AD03-11 | 1.1 | 1.10 | 1.4 | 4.0 | 0.7 | 1.2 | 5.8 | 0.9 | 1.8 | 9.1 |
| AD03-12 | 1.1 | 1.12 | 1.8 | 4.6 | 0.7 | 1.1 | 6.1 | 0.9 | 1.8 | 10.2 |
| AD03-13 | 1.1 | 1.16 | 1.8 | 5.6 | 0.7 | 1.4 | 7.8 | 0.9 | 2.2 | 12.2 |
| AD03-14 | 1.2 | 1.13 | 1.9 | 5.8 | 0.7 | 1.5 | 8.0 | 0.9 | 2.3 | 12.5 |
| AD03-15 | 1.1 | 1.09 | 1.9 | 4.9 | 0.7 | 1.2 | 7.3 | 0.8 | 2.0 | 12.3 |
| AD03-16 | 1.2 | 1.15 | 1.3 | 3.0 | 0.7 | 1.0 | 5.0 | 1.0 | 1.7 | 11.3 |

| | | | | | | | | | | |
|---|---|---|---|---|---|---|---|---|---|---|
| Compositions AD03-3 and AD03-6 were not assayed because precipitates formed immediately after preparation of the compositions. | | | | | | | | | | |

## Claims

1. A pharmaceutical formulation containing dalbavancin or a pharmaceutically acceptable salt thereof, a cyclodextrin and water.

2. The formulation according to claim 1, wherein the cyclodextrin is sulfobutyl ether beta-cyclodextrin or a sodium salt thereof, and the molar ratio of dalbavancin to cyclodextrin is within the range of from 1:2.5 to 1:6, even more preferably 1:3 to 1:5.

3. The formulation according to claim 1, wherein the cyclodextrin is hydroxypropyl beta-cyclodextrin, and the molar ratio of dalbavancin to cyclodextrin is within the range of from 1:0.1 to 1:6, more preferably 1:1 to 1:5.

4. The formulation according to any one of the preceding claims, which has a pH within the range of 3 to 5.5.

5. The formulation according to any one of the preceding claims, which has a pH about 4.5.

6. The formulation according to any one of the preceding claims, which contains at least 95 % of the initial amount of dalbavancin or a pharmaceutically acceptable salt thereof after 1 month storage at 25 °C, and at least 90 %, preferably at least 95 % of the initial amount of dalbavancin or a pharmaceutically acceptable salt thereof after 3 months storage at 25 °C.

7. The formulation according to any one of the preceding claims, which contains at least 90 % of the initial amount of dalbavancin or a pharmaceutically acceptable salt thereof after 1 month storage at 40 °C, and at least 75 % of the initial amount of dalbavancin or a pharmaceutically acceptable salt thereof after 3 months storage at 40 °C.

8. The formulation according to any one of the preceding claims, which contains at least 97 % of the initial amount of dalbavancin or a pharmaceutically acceptable salt thereof after 1 month storage at 5 °C, and at least 96 % of the initial amount of dalbavancin or a pharmaceutically acceptable salt thereof after 3 months storage at 5 °C.

9. The formulation according to any one of the preceding claims, which is free of sugars and sugar alcohols.

10. The formulation according to any one of the preceding claims, which is free of mannitol and lactose.

11. The formulation according to any one of the preceding claims, which is in the form of a concentrate for an infusion solution or parenteral solution, or in the form of an infusion solution or a parenteral solution.

12. The formulation according to any one of the preceding claims, which contains dalbavancin or a pharmaceutically acceptable salt thereof in a concentration of 0.1 to 100 mg per ml of final solution, preferably 0.5 to 50 mg per ml of final solution, or 1 to 10 mg per ml of final solution.

13. The formulation according to any one of the preceding claims for use in prevention a bacterial infection or in the treatment of a bacterial infection, in particular in the prevention or treatment of an infection caused by *Staphylococcus, Neisseria, Clostridium,* or *Streptococcus* species.

14. The formulation for use according to claim 13, wherein the formulation is co-administered simultaneously or sequentially with another antibiotic.

15. The formulation according to any one of the claims 1 to 9 for use in the treatment of acute osteomyelitis in children.
